# EUROPEAN PATENT APPLICATION

(11) **EP 4 591 913 A2**
(43) Date of publication of application: **30.07.2025**
(21) Application number: 25182009.8
(22) Date of filing: 26.01.2023
(51) Int. Cl.: A61M 25/06

(54) **EXPANDABLE INTRODUCER**

(30) Priority: 27.01.2022 US 202263303620 P; 13.07.2022 US 202263388728 P
(62) Divisional of application: 23707617.9
(71) Applicant: Medtronic, Inc., Minneapolis, Minnesota 55432 (US)
(72) Inventor: ANDERSON, Marc, A., Galway (IE); FARRELL, Timothy, Desmond, Galway (IE); MORELLI, Fionnuala, Galway (IE); SCIACCHITANO, Maro, J., Galway (IE); NORGROVE, Matthew, P., Galway (IE); CLARKE, Luke, A., Galway (IE); REID, Mark, Desmond, Galway (IE); COULTRY, Laura, Marie, Galway (IE); SAIL, Shridhar, R., Galway (IE); MCGUINN, Alan, Thomas, Galway (IE); CASLEY, Mark, Santa Rosa (US); DESHMUKH, Susheel, R., Santa Rosa (US); OGORMAN, Jacqueline, Bernadette, Galway (IE); MCAFEE, Patricia, Galway (IE); MORAN, Chris, Galway (IE); TROTTA, Antonia, Galway (IE)
(74) Representative: Zimmermann & Partner Patentanwälte mbB

(57) **Abstract**

An expandable introducer includes a hub and a tubular sheath coupled to the hub and extending distally from a distal end thereof. The tubular sheath defines a central lumen. A circumference of the tubular sheath includes a full thickness region having a first wall thickness and a reduced thickness region having a second wall thickness smaller than the first wall thickness. The reduced thickness region is configured to be folded such that the tubular sheath includes an unexpanded, folded state and is configured to expand to an expanded, unfolded state in response to a device passing though the central lumen. A first outer diameter of the tubular sheath in the unexpanded, folded state is smaller than a second outer diameter of the tubular sheath in the expanded, unfolded state.

## Description

### FIELD OF THE INVENTION

The present invention relates to an expandable introducer device for providing percutaneous access to a patient's vasculature for a transcatheter device delivery system

### BACKGROUND

An introducer device is used to provide percutaneous access to the vascular system of a patient and functions to permit the introduction and positioning of various minimally invasive delivery devices within the patient's vasculature. In general, making the smallest incision is the most desirable and leads to less complications, less trauma, and improved patient outcomes. Some delivery devices, however, are large and require large sheaths to accommodate and help deliver them to the desired site within the body.

### BRIEF SUMMARY OF THE INVENTION

In accordance with a first example hereof, an expandable introducer includes a hub having a distal end and a proximal end, and a tubular sheath coupled to the hub and extending distally therefrom. The tubular sheath defines a central lumen. A circumference of the tubular sheath includes a thick wall region having a first wall thickness and a thin wall region having a second wall thickness smaller than the first wall thickness. The thin wall region is configured to be folded such that the tubular sheath includes an unexpanded, folded state and is configured to expand to an expanded, unfolded state in response to a device passing though the central lumen. A first outer diameter of the tubular sheath in the unexpanded, folded state is smaller than a second outer diameter of the tubular sheath in the expanded, unfolded state.

In a second example, in the expandable introducer of the first example, the first wall thickness is about 0.4-0.7mm.

In a third example, in the expandable introducer of the first example or the second example, the second wall thickness is about 0.05-0.2mm.

In a fourth example, in the expandable introducer of any one of first through third examples, the thick wall region of the tubular sheath includes an outer wall having a first layer and a second layer, and a liner coupled to an inner surface of the outer wall, the liner defining the central lumen of the tubular sheath, and the thin wall region of the tubular sheath includes the liner and only the second layer of the outer wall.

In a fifth example, in the expandable introducer of the fourth example, the second layer in the thick wall region has a third thickness, and the second layer in the thin wall region has a fourth thickness smaller than the third thickness.

In a sixth example, in the expandable introducer of any one of the first through fifth examples, the tubular sheath comprises a proximal end, wherein the proximal end of the tubular sheath does not include an unexpanded, folded configuration.

In a seventh example, the expandable introducer of any one of the first through sixth examples further includes a hemostatic jacket surrounding at least a proximal end of the tubular sheath, wherein the hemostatic jacket is configured to prevent blood flow from outside of the tubular sheath.

In an eighth example, in the expandable introducer of the seventh example, the hemostatic jacket is radially expandable.

In a ninth example, in the expandable introducer of the seventh example or the eighth example, hemostatic jacket is about 10 - 12 cm in length.

In a tenth example, in the expandable introducer of the seventh example or the eighth example, the hemostatic jacket is about 30 - 35 cm in length.

In an eleventh aspect, in the expandable introducer of any one of the seventh through tenth aspects, the hemostatic jacket is heat bonded to the proximal end of the tubular sheath.

In a twelfth example, in the expandable introducer of any one of the seventh through eleventh examples, the hemostatic jacket includes an angled tip.

In a thirteenth example, in the expandable introducer of any one of the seventh through twelfth examples, the hemostatic jacket is attached to only the thick wall region of the tubular sheath.

In a fourteenth example, the expandable introducer of any one of the first through thirteenth examples further includes a tip coupled to a distal end of the tubular sheath and extending distally from the distal end of the tubular sheath.

In a fifteenth example, in the expandable introducer of the fourteenth example, the tip comprises a weakened region configured to tear to allow the tip to radially expand in response to a device being advanced through the tip.

In a sixteenth example, in the expandable introducer of the fifteenth example, the tip includes a axial slit, wherein circumferential edges at proximal and distal ends of the longitudinal are attached to each other, and wherein the weakened region comprises the axial slit between the proximal and distal attachments.

In a seventeenth example, in the expandable introducer of the fifteenth example, the weakened region comprises a longitudinal perforation.

In an eighteenth example, in the expandable introducer of any one of the first through seventeenth examples, the tubular sheath is shape set to return to the unexpanded, folded state after the device has passed through to the central lumen.

In a nineteenth example, in the expandable introducer of any one of the first through eighteenth examples, the first outer diameter of the tubular sheath is about 5-7mm.

In a twentieth example, in the expandable introducer of any one of the first through nineteenth examples, the second outer diameter of the tubular sheath is about 8-10mm.

In a twenty-first example, an introducer hub includes a hub body having a proximal end, a distal end, and a central lumen, a seal section disposed within the central lumen of the hub body, and a sealcap coupled to the hub body. The seal section includes at least one seal. The sealcap is at least partially disposed within the central lumen. The sealcap includes a distal end abutting the seal section, and a locking mechanism on an internal surface thereof configured to interlock with a device to be inserted into the introducer hub.

In a twenty-second example, in the introducer hub of the twenty-first example, the locking mechanism includes in internal quarter-turn thread, a haptic feedback bump, and a hard-stop.

In a twenty-third example, in the introducer hub of the twenty-first example of the twenty-second example, the sealcap includes a plurality of protrusions extending from an outer surface thereof, the hub body includes a plurality of openings, and the plurality of protrusions are configured to mate with plurality of openings to couple the sealcap to the hub body in a snap-fit connection.

In a twenty-fourth example, in the introducer hub of the twenty-third example, the plurality of protrusions are disposed on the proximal end of the sealcap and the plurality of openings are disposed on the proximal end of the hub body.

In a twenty-fifth example, in the introducer hub of any one of the twenty-first through twenty-fourth examples, the sealcap has a longitudinal length of about 14-16 mm, and internal diameter of about 19-22mm, and wherein a maximum insertion angle of a shaft that can pass through the seal section is 5-6°.

In a twenty-sixth example, in the introducer hub of any one of the twenty-first through twenty-fifth examples, the seal section comprises a first seal, a second seal, and a third seal.

In a twenty-seventh example, in the introducer hub of the twenty-sixth example, the first seal comprises a central passageway that is substantially conically-shaped tapering from a proximal end of the first seal to a substantially circular hole extending through a distal end of the first seal.

In a twenty-eighth example, in the introducer hub of the twenty-sixth example or the twenty-seventh example, the second seal is disposed distal to the first seal and comprises a slit including a central portion that aligns with the hole of the first seal, wherein the slit enables the device to be inserted into the introducer hub extend through the second seal while maintaining hemostasis by sealing around an exterior surface of the device.

In a twenty-ninth example, in the introducer hub of any one of the twenty-sixth through twenty-eighth examples, the third seal is disposed distal of the second seal and comprises a central opening, a slit, and a cavity.

In a thirtieth example, in the introducer hub of the twenty-ninth example, the third seal a distal surface and a proximal surface opposite the distal surface, wherein the central opening extends from the proximal surface to the distal surface and the slit extends from the proximal surface to the distal surface.

In a thirty-first example, in the introducer hub of the thirtieth example, the slit of the third seal is perpendicular to the slit of the second seal.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF DRAWINGS

The foregoing and other features and advantages of the present disclosure will be apparent from the following description of embodiments hereof as illustrated in the accompanying drawings. The accompanying drawings, which are incorporated herein and form a part of the specification, further serve to explain the principles of the present disclosure and to enable a person skilled in the pertinent art to make and use the embodiments of the present disclosure. The drawings may not be to scale.
FIG. 1 shows a perspective side view of an expandable introducer according to an embodiment hereof.
FIG. 2A shows a cross-section of the tubular sheath in an unexpanded, folded state according to an embodiment hereof.
FIG. 2B shows a close-up view of a section of the tubular sheath of FIG. 2A.
FIG. 2C shows a cross-section of the tubular sheath in an expanded, unfolded state according to an embodiment hereof.
FIG. 2D shows a schematic cross-section of the tubular sheath in the expanded, unfolded state according to an embodiment hereof.
FIG. 2E shows a schematic cross-section of another embodiment of the tubular sheath in the expanded, unfolded state.
FIG. 2F shows a schematic cross-section of another embodiment of the tubular sheath in the expanded, unfolded state.
FIG. 3A shows the expandable introducer according to an embodiment hereof.
FIG. 3B shows a close-up view of a tip of the expandable introducer according to an embodiment hereof.
FIG. 3C shows a close-up view of the tip of FIG. 3B after a device has been advanced therethrough according to an embodiment hereof.
FIG. 3D shows a close-up view of the tip of the expandable introducer according to an embodiment hereof.
FIG. 3E shows a close-up view of the tip of FIG. 3D after a device has been advanced therethrough according to an embodiment hereof.
FIG. 4A shows a side view of the expandable introducer according to an embodiment hereof.
FIG. 4B shows a hemostatic jacket of the expandable introducer of FIG. 4A.
FIG. 4C shows a close-up view of the hemostatic jacket of FIG. 4B.
FIG. 5A shows an exploded side view of the introducer hub and sealcap according to an embodiment hereof.
FIG. 5B shows a side cross-sectional view of the introducer hub and the sealcap of FIG. 5A.
FIG. 6 shows a side cross-sectional view of the introducer hub according to an embodiment hereof.
FIG. 7 shows a lateral cross-sectional view of a portion of the introducer hub.
FIG. 8A shows a side cross-sectional view of the introducer hub, sealcap, and seal section according to an embodiment hereof.
FIG. 8B shows a close-up view of the seal section of FIG. 8A,
FIG. 9A shows a proximal view of a first seal according to an embodiment hereof.
FIG. 9B shows a side cross-sectional view of the first seal of FIG. 9A.
FIG. 9C shows a distal view of the first seal of FIG. 9A.
FIG. 10A shows a proximal view of a second seal according to an embodiment hereof.
FIG. 10B shows a side cross-sectional view of the second seal of FIG. 10A.
FIG. 10C shows a distal view of the second seal of FIG. 10A.
FIG. 11A shows a proximal view of a third seal according to an embodiment hereof.
FIG. 11B shows a side cross-sectional view of the third seal of FIG. 11A.
FIG. 11C shows a distal view of the third seal of FIG. 11A.
FIG. 12A shows a side view of the introducer hub and a valve relief.
FIG. 12B shows a side cross-sectional view of the introducer hub and valve relief of FIG. 12A.
FIG. 13 shows the expandable introducer in use with a dilator assembly according to an embodiment hereof
FIG. 14A shows a side view of a dilator hub of the dilator assembly.
FIG. 14B shows a distal end view of the dilator hub of FIG. 14A.
FIG. 15 shows a side view of the dilator hub coupled to the introducer hub.
FIG. 16A shows a side cross-sectional view of the introducer hub and a side view of the dilator hub according to embodiments hereof.
FIG. 16B shows a side cross-sectional view of the dilator hub coupled to the introducer hub according to an embodiments hereof.
FIG. 16C shows a lateral cross-sectional view of the dilator hub coupled to the introducer hub according to an embodiments hereof.

### DETAILED DESCRIPTION

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single device or component for purposes of clarity, it should be understood that the techniques of this disclosure may be performed by a combination of devices or components associated with, for example, a delivery device. The following detailed description is merely exemplary in nature and is not intended to limit the invention of the application and uses of the invention. Furthermore, there is no intention to be bound by any expressed or implied theory presented in the preceding field of the invention, background, summary or the following detailed description.

As used in this specification, the singular forms "a", "an" and "the" specifically also encompass the plural forms of the terms to which they refer, unless the content clearly dictates otherwise. The term "about" is used herein to mean approximately, in the region of, roughly, or around. When the term "about" is used in conjunction with a numerical range, it modifies that range by extending the boundaries above and below the numerical values set forth. In general, the term "about" is used herein to modify a numerical value above and below the stated value by a variance of 20%. It should be understood that use of the term "about" also includes the specifically recited number of value.

The terms "proximal" and "distal" herein are used with reference to the clinician using the devices. Therefore, "proximal" and "proximally" mean in the direction toward the clinician, and "distal" and "distally" mean in the direction away from the clinician. In other words, "proximal" and "proximally" mean towards the hub end of the introducer and "distal" and "distally" means toward the tip end of the introducer.

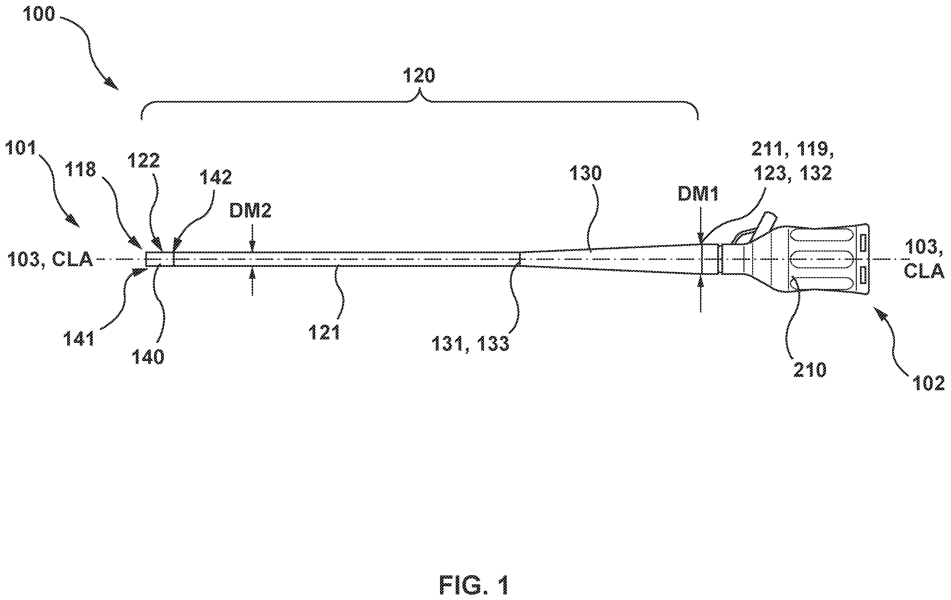

FIG. 1 shows an expandable introducer 100 according to embodiments herein. The expandable introducer 100 includes a distal end 101 and a proximal end 102. The expandable introducer 100 further includes an introducer hub 210 and a shaft portion 120. The introducer hub 210 includes a distal end 211 and a proximal end 212. The shaft portion 120 of the expandable introducer 100 includes a distal end 118 and proximal end 119. The proximal end 119 of the shaft portion 120 is coupled to the distal end 211 of the introducer hub 210 and extends distally therefrom. The expandable introducer 100 includes a central lumen or passageway 103 that runs parallel to a central longitudinal axis CLA, the central lumen 103 extending an entire longitudinal length of the expandable introducer 100, from a distal end 101 of the expandable introducer 100 to the proximal end 102 of the expandable introducer 100. The central passageway 103 of the expandable introducer 100 is sized and shaped to accommodate a device (not shown) therethrough, as described in more detail below.

As shown in FIG. 1, the shaft portion 120 further includes a tubular sheath 121, a hemostatic jacket 130 and a tip 140. The tubular sheath 121 includes a distal end 122 and a proximal end 123. The hemostatic jacket 130 encloses, or covers, the proximal end 123 of the tubular sheath 121 and the tip 140 encloses, or covers, the distal end 122 of the tubular sheath 121, as described in more detail below. The shaft portion 120 of the expandable introducer 100 tapers in a distal direction when the tubular sheath is in a folded state, which is described in detail below, such that a proximal diameter DM1 of the shaft portion 120 at the proximal end 119 is greater than a distal diameter DM2 of the shaft portion 120 at the distal end 118, as best shown in FIG. 1. The proximal diameter DM1 of the shaft portion 120 at the proximal end 119 is about 8.0 -10.0 mm and the distal diameter DM2 of the shaft portion 120 at the distal end 118 is about 5.0-7.0 mm.

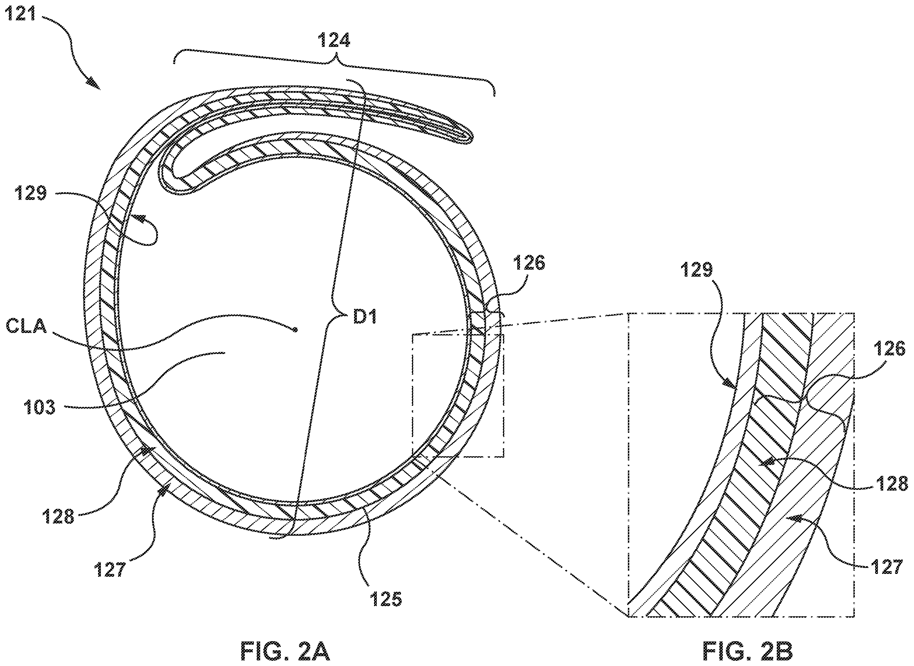

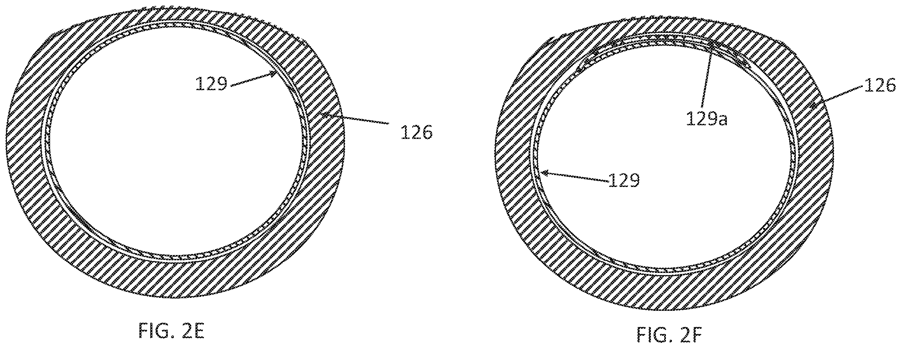

The proximal end 123 of the tubular sheath 121 is coupled to the distal end 211 of the introducer hub 210 and extends distally and longitudinally therefrom, as shown in FIG. 1. Referring to FIGS. 2A-2D, the tubular sheath 121 includes an outer wall 126 and a liner 129. The outer wall 126 and the liner 129 are tubular structures that make up the tubular sheath 121. The liner 129 is disposed inside of and coupled to an interior surface of the outer wall 126. The outer wall 126 of the tubular sheath 121 is a variable wall thickness layer. In the embodiment of FIGS. 2A-2D the outer wall includes a first layer 127 and a second layer 128. In other embodiments, as shown in FIG. 2E, the outer wall 126 is a single, variable thickness layer. In another embodiment, as shown in FIG. 2F, the liner 129 may include a reinforcement layer 129A for resistance to tear/puncture when the delivery system is advanced therethrough. The reinforcement layer 129A may be a layer attached to the outer surface of the liner 129 or may be formed integral with the liner 129. In the embodiment of FIGS. 2A-2D, the first layer 127 of the outer wall 126 is the radially outer-most layer of the outer wall 126 and the second layer 128 of the outer wall 126 is disposed radially inward in relation to the first layer 127 of the outer wall 126, as best shown in FIGS. 2A-2B. Thus, the liner 129 of the tubular sheath 121 is disposed inside of and coupled to an interior surface of the second layer 128 of the outer wall 126 such that the liner 129 is the radially innermost layer of the tubular sheath 121 and defines the central lumen 103 of the tubular sheath 121, as best shown in FIG. 2B. The first layer 127, second layer 128, and liner 129 may be formed by co-extrusion or a layered reflow lamination processes. In the embodiment shown, the first layer 127 of the outer wall 126 comprises polyether block amine (PEBAX) with a Shore D durometer value of about 63-74D. The second layer 128 of the outer wall 126 comprises PEBAX with a Shore D durometer value of about 40-63D. In an embodiment, the second layer 128 comprises PEBAX with a Shore D durometer value of about 40D. Those skilled in the art will recognize that the features of the different embodiments described with respect to FIGS. 2A-2E may be interchanged. For example, and not by way of limitation, the reinforcement layer 129A may be utilized with an outer wall that includes two layers.

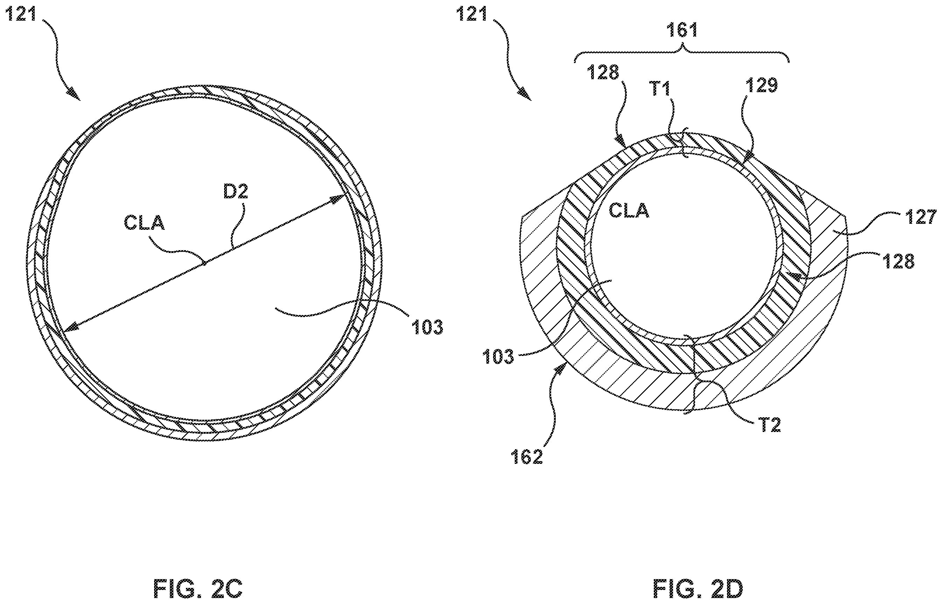

The liner 129 of the tubular sheath 121, as best shown in FIGS. 2B and 2D, is a thin, difficult to pierce, low-friction plastic tube disposed within and coupled to the interior surface of the outer wall 126 of the tubular sheath 121. The liner 129 is configured to reduce friction forces and aid in the translation of the device through the central lumen 103 of the tubular sheath 121 as the device is advanced distally towards the distal end 101 of the expandable introducer 100. The thickness of the liner 129 is about 0.05-0.15 mm and is constant, or continuous, throughout the entire tubular sheath 121 of the shaft portion 120. The liner 129 may be polytetrafluoroethylene (PTFE), fluorinated ethylene propylene (FEP) or other materials known to those skilled in the art suitable for the purposes described herein, or any combination thereof.

The tubular sheath 121 includes an unexpanded, folded state and an expanded, unfolded state. The tubular sheath 121 maintains the unexpanded, folded state until a device is advanced through the tubular sheath 121, during which the tubular sheath 121 is configured to expand to the expanded, unfolded state, which is described in more detail below. When the tubular sheath 121 is in the unexpanded, folded state, the tubular sheath 121 includes a folded region 124 and a non-folded region 125, as shown in FIG. 2A. The fold region 124 is defined as the region where a section of the tubular sheath 121 is folded over, as can be seen in FIG. 2A. In other words, a portion of the tubular sheath 121 is folded and bent to overlap with itself. The non-folded region 125 of the tubular sheath 121 is defined as the remainder of the tubular sheath 121 that does not include a fold. As can be seen in FIG. 2A, when folded, the tubular sheath 121 includes two folds or bends.

The tubular sheath 121 has a varying thickness around a circumference of the tubular sheath 121. In particular, as best shown in FIG. 2D, the tubular sheath 121 includes a thin wall region 161 having a minimum first wall thickness T1. The tubular sheath 121 further includes a thick wall region 162 having second wall thickness T2 that is greater than the first wall thickness T1. In the embodiment shown, the thick wall region 162 is the remainder of the circumference of the tubular sheath 121 that is not the thin wall region 161. The first wall thickness T1 of the tubular sheath 121 of the thin wall region 161 is reduced by gradually removing the first layer 127 of the outer wall 126 to transition from the second wall thickness T2 of the thick wall region 162 to the first wall thickness T1 of the thin wall region 161, as shown in FIG. 2D. In other words, the second wall thickness T2 of the tubular sheath 121 gradually decreases equally around the circumference of the tubular sheath 121 until the first wall thickness T1 is achieved at the thin wall region 161. Thus, the tubular sheath 121 in the thin wall region 161 includes only the second layer 128 of the outer wall 126 and the liner 129 of the tubular sheath 121, as can be seen in FIG. 2D. The tubular sheath 121 at the thick wall region 162 includes the first layer 127 of the outer wall 126, the second layer 128 of the outer wall 126, and the liner 129. As shown in FIG. 2D, the thickness of the second layer 128 of the outer wall 126 in the thin wall region 161 is equal to the thickness of the second layer 128 of the outer wall 126 in the thick wall region 162. The first wall thickness T1 of the tubular sheath 121 at the thin wall region 161 is reduced compared to the second wall thickness T2 of the tubular sheath 121 at the thick wall region 162, thereby enabling the tubular sheath 121 to be folded into the folded state shown in FIG. 2A. In the folded state, the tubular sheath 121 has a first outer diameter D1 that is smaller than a second outer diameter of the tubular sheath 121 in the unfolded state, as described below Although the thin wall region 161 in this embodiment includes a full thickness of the second layer 128 of the outer wall 126, in other embodiments, the thin wall region 161 may include a reduced thickness of the second layer 128, thereby having the thickness reduced by removal of the first layer 127 from the outer wall 126 and reducing the thickness of the second layer 128. Gradually removing the first layer 127, as shown in FIG. 2D, reduces insertion forces necessary when inserting the expandable introducer 100 into the vasculature of the patient. The thickness T1 of the wall of the tubular sheath 121 in the thin wall region 161 is about 0.05-0.15 mm and the thickness T2 of the wall of the tubular sheath 121 in the thick wall region 162 is about 0.4-0.7mm.

The tubular sheath 121 is configured to expand radially from the unexpanded, folded state having the first outer diameter D1 to the expanded, unfolded state having a second outer diameter D2 larger than the first outer diameter D1 in response to a device being advanced therethrough. As the device is advanced through the tubular sheath 121 of the shaft portion 120 of the expandable introducer 100, the folded region 124 of the tubular sheath 121 is configured to unfold such that the tubular sheath 121 may expand to the second outer diameter D2 of the expanded, unfolded state, shown in FIG. 2C. When the tubular sheath 121 is in the expanded, unfolded state, there is no longer a folded region 124. In other words, the tubular sheath 121 in the expanded, unfolded state has a substantially circular-shaped cross-section, as can be seen in FIG. 2C. The first diameter D1 of the tubular sheath 121 in the unexpanded, folded state is about 5-7mm and the second diameter D2 of the tubular sheath 121 in the expanded, unfolded state is about 8-10 mm.

The tubular sheath 121 is shape-set such that the tubular sheath 121 may shape-set back to the folded state after the device has advanced through the tubular sheath 121. More specifically, the reduced first thickness T1 of the folded region 124 of the tubular sheath 121 allows the tubular sheath 121 to return to the unexpanded, folded state autonomously after the tubular sheath 121 has been expanded by the device. In both the expanded and unexpanded states, the proximal-most end 123 of the tubular sheath 121, where the tubular sheath 121 couples to the circular-shaped distal end 211 of the introducer hub 210, does not include the fold in the tubular sheath 121. In other words, the fold does not extend to the proximal-most end 123 of the tubular sheath 121.

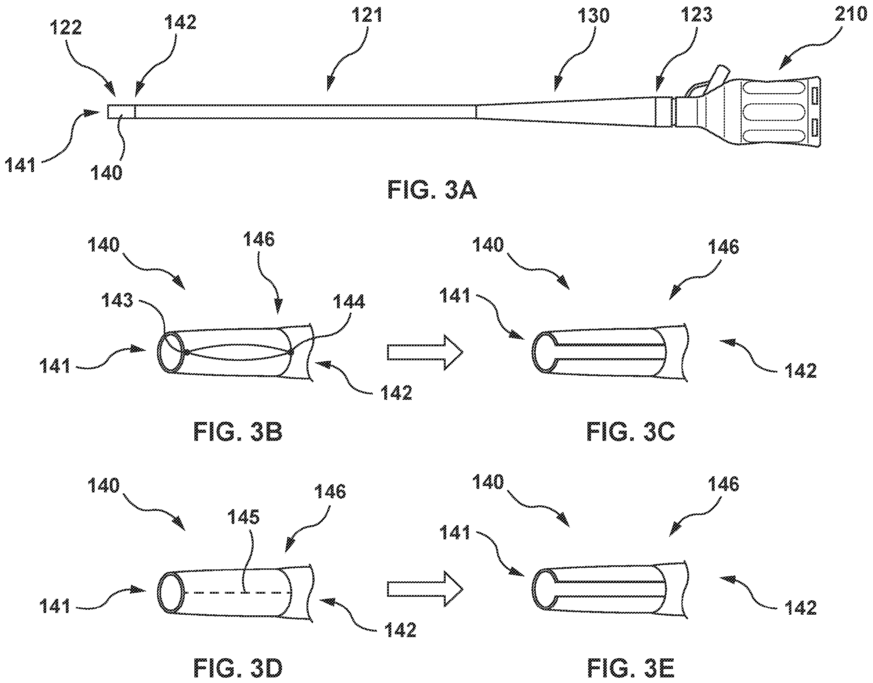

FIG. 3A shows the expandable introducer 100 according to embodiments herein. The tip 140 of the expandable introducer 100, shown in FIGS. 3A-3E, is a tubular structure that includes a distal end 141 and a proximal end 142. The proximal end 142 of the tip 140 couples to an outer surface of the distal end 122 of the tubular sheath 121 and extends distally therefrom. The proximal end 142 of the tip 140 is coupled to an outer surface of the tubular sheath 121 such that the tip 140 covers, or overlaps with, the distal end 122 of the tubular sheath 121. The tip 140 enables a smooth insertion transition at the distal end of the tubular sheath 121. In particular, without the tip 140, the abrupt surface of the tubular sheath 121 with the folded region 124 in the unexpanded, folded state, as shown in FIG. 2A, would be inserted into the vessel of a patient. Such an abrupt surface may cause damage to the vessel. The proximal end 142 of the tip 140 is bonded to, or coupled to, the outer surface of the tubular sheath 121 by melt reflow of the proximal end 142 of the tip 140 with the distal end 122 of the tubular sheath 121. The proximal end 142 of the tip 140 may also be coupled to the distal end 122 of the tubular sheath by fusing or reflow processes. The tip 140 gradually tapers in a distal direction such that a diameter of the proximal end 142 of the tip 140 is larger than a diameter of the distal end 141 of the tip 140. The tip 140 may comprise polyether block amide (PEBAX), Polyurathatie or other suitable thermoplastic polymer, and/or any combination thereof.

FIGS. 3B-3C show an embodiment of the tip 140 of the expandable introducer 100 of FIG. 3A. Prior to connection to the tubular sheath 121, the tip 140 is generally tubular with an axial slit extending from the proximal end 142 to the distal end 141 of the tip 140. In other embodiments, the tip 140 may be a substantially rectangular piece of material that is bent into a tubular shape with the edges of the material not attached, thereby creating a tubular shape with the axial slit. As explained above, the proximal end 142 is attached to the distal end 122 of the tubular sheath 121. Further, in the embodiment shown in FIGS. 3B-3C, a first connection point 143 at the distal end 141 of the tip 140 connects the circumferential edges of the axial slit, and a second connection point 144 at the proximal end 142 of the tip connects the circumferential edges of the axial slit. The first and second connection points 143, 144 of the tip 140 may be aligned and such that they are substantially parallel to the central longitudinal axis CLA of the expandable introducer 100, as shown in FIG. 3B. Between the first and second connection points 143, 144, the circumferential edges of the axial slit are not connected to each other. The unconnected portion between the first and second connection points 143, 144 leaves a weakened region. Thus, the tip 140 provides a smooth transition from the folded region 124 of the tubular sheath 121 and enables a smooth insertion into the vessel. When the device is advanced through the tubular sheath 121 through the tip 140, the first and second connection points 143, 144 of the tip 140 are configured to split, or break, as the device is advanced therethrough. FIG. 3C shows the tip 140 of FIG. 3B after the device has been advanced through the tip 140 and the first and second connection points 143, 144 have been broken. This enables the distal end 122 tubular sheath 121 to unfold and radially expand, and enables the tip 140 to radially expand to enable the device to extend therethrough. Once the connection points 143, 144 have been broken, the tip 140 is no longer a closed, tubular shape and the opening, or split, extends the entire longitudinal length of the tip 140, from the distal end 141 to the proximal end 142 of the tip 140. The tip 140 remains attached to the distal end 122 to the tubular sheath 121.

FIGS. 3D-3E show another embodiment of the tip 140 of the expandable introducer 100 of FIG. 3A. As with the embodiment above, prior to connection to the tubular sheath 121, the tip 140 may be generally tubular with an axial slit extending from the proximal end 142 to the distal end 141 of the tip 140. In other embodiments, the tip 140 may be a substantially rectangular piece of material that is bent into a tubular shape with the edges of the material not attached, thereby creating a tubular shape with the axial slit. As explained above, the proximal end 142 is attached to the distal end 122 of the tubular sheath 121. Further, in the embodiment shown in FIGS. 3D-3E, the circumferential edges of the axial slit are connected to each other along substantially the entire length of the tip 140. The connection may be substantially parallel to the central longitudinal axis CLA of the expandable introducer 100, as shown in FIG. 3D. A perforation may then be provided at the connection between the circumferential edges, thereby creating a perforated joint 145. The perforated joint 145 leaves a weakened region. Thus, the tip 140 provides a smooth transition from the folded region 124 of the tubular sheath 121 and enables a smooth insertion into the vessel. When the device is advanced through the tubular sheath 121 through the tip 140, the perforated joint 145 is configured to split, or break, as the device is advanced therethrough. FIG. 3E shows the tip 140 of FIG. 3D after the device has been advanced through the tip 140 and the perforated joint 145 has been broken. This enables the distal end 122 tubular sheath 121 to unfold and radially expand, and enables the tip 140 to radially expand to enable the device to extend therethrough. Once the perforated joint 145 has been broken, the tip 140 is no longer a closed, tubular shape and the opening, or split, extends the entire longitudinal length of the tip 140, from the distal end 141 to the proximal end 142 of the tip 140. The tip 140 remains attached to the distal end 122 to the tubular sheath 121.

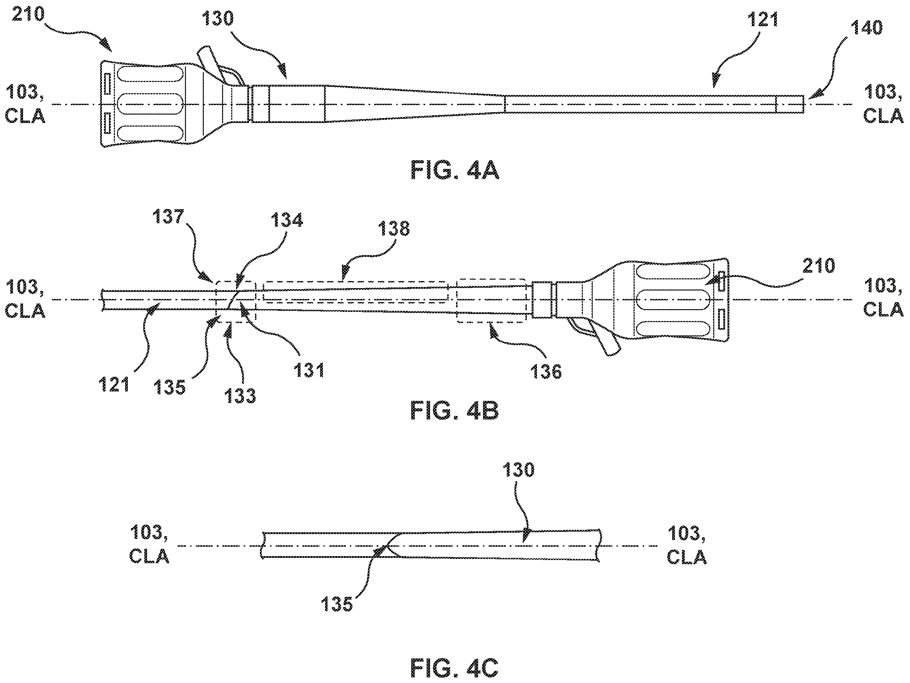

FIG. 4A shows a close-up view of the hemostatic jacket 130 of the introducer according to embodiments herein. The hemostatic jacket 130 may be a tubular structure that includes a distal end 131 and a proximal end 132. A proximal portion of the tubular sheath 121 is disposed within a central lumen of the hemostatic jacket 130. In other words, the hemostatic jacket 130 is disposed radially outward in relation to the tubular sheath 121 of the shaft portion 120 of the introducer 100. As shown in FIG. 4B, the hemostatic jacket 130 may be coupled to the tubular sheath 121 by heat bonding at the proximal and distal ends thereof. At the proximal end 132, the hemostatic jacket 130 is bonded at a proximal bond 136 around a full circumference thereof to the proximal end 122 of the tubular sheath 121 that does not include a folded region 124. The proximal bond 136 may be via applied heat to meld the materials of the hemostatic jacket 130 and the tubular sheath 121 together, or other bonds suitable for the purposes hereof. The proximal bond 136 provides a hemostatic seal such that blood does not pass proximally through the folded region 124 of the tubular sheath 121. At a distal bond 137, the hemostatic jacket 130 may be bonded to the tubular sheath 121 along the unfolded region 125 thereof to enable the folded region 124 to unfold. In an unbonded region 138, the hemostatic sheath 130 may be unbonded to the tubular sheath 121 or may be bonded to the tubular sheath along only the unfolded region 125 thereof. The hemostatic jacked 130 is radially expanded such that it radially expands when the device is advanced through the tubular sheath 121. The hemostatic jacket 130 may comprise ePTFE, low durometer PEBAX (about 25D), polyurethane, and/or other expandable materials, and/or any combinations thereof Such material allows the hemostatic jacket 130 to be low stiffness and minimizes the force needed to expand the tubular sheath 121. Such material may also be processed to minimize the coefficient of friction between the hemostasis jacket 130 and the sheath shaft.

In some embodiments, the hemostatic jacket 130 may be about 10-12 cm in length. In other embodiments, the hemostatic jacket 130 may be about 30-35 cm in length. However, these example lengths are not meant to be limiting, and the hemostatic jacket 130 may be of any length suitable for the purposes described herein.

In an embodiment shown in FIGS. 4B and 4C, hemostatic jacket 130 further includes an angled cut 133 at the distal end 131. The angled cut 133 includes a first side 134 and a second side 135 approximately 180 degrees apart from the first side 134. In other words, the first side 134 and the second side 135 of the angled cut 133 are disposed on opposite sides of the distal end 131 of the hemostatic jacket 130. The first side 134 of the angled cut 133 terminates proximal in relation to the second side 135 of the angled cut 133. In other words, the second side 135 of the angled cut 133 extends further distally compared to the first side 134 of the angled cut 133. The first side 134 of the angled cut 133 aligns with the folded region 124 of the tubular sheath 121 and is not bonded to the folded region 124 of the tubular sheath 121. The second side 135 of the angled cut 133 aligns with the non-folded region 125 of the tubular sheath 121 and is bonded to the non-folded region 125 of the tubular sheath 121. The angled cut 133 increases the surface area of the hemostatic jacket 130 for attachment to the tubular sheath 121. Further, the second side 135 of the angled cut 133 is configured to act as a lead-in for the first side 134 of the angled cut 133 and the region of the hemostatic jacket 130 that is unbonded to the folded region 124 of the tubular sheath 121. FIG. 4C shows a close-up view of the second side 135 of the angled cut 133 disposed at the distal end 131 of the hemostatic jacket 130. As can be seen, the angled cut 133 creates a triangular-shaped point at the second side 135 of the angled cut 133 that aligns with the non-folded region 125 of the tubular sheath 121. The angle of the angled cut can range from 30-50 degrees relative to the central longitudinal axis CLA. In other words, the angle from the first side 134 of the angled cut to the second side 135 of the angled cut with respect to the central longitudinal axis CLA is approximately 30-50 degrees.

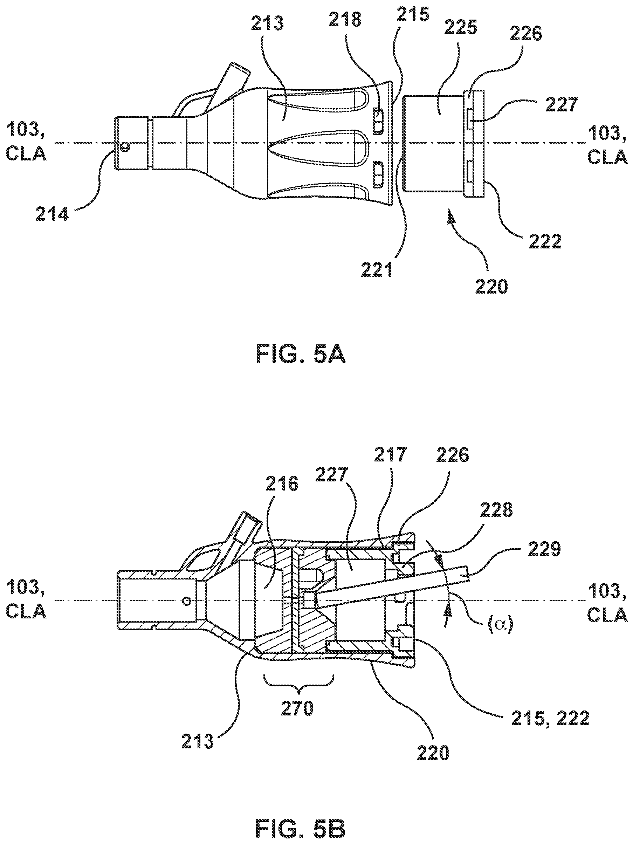

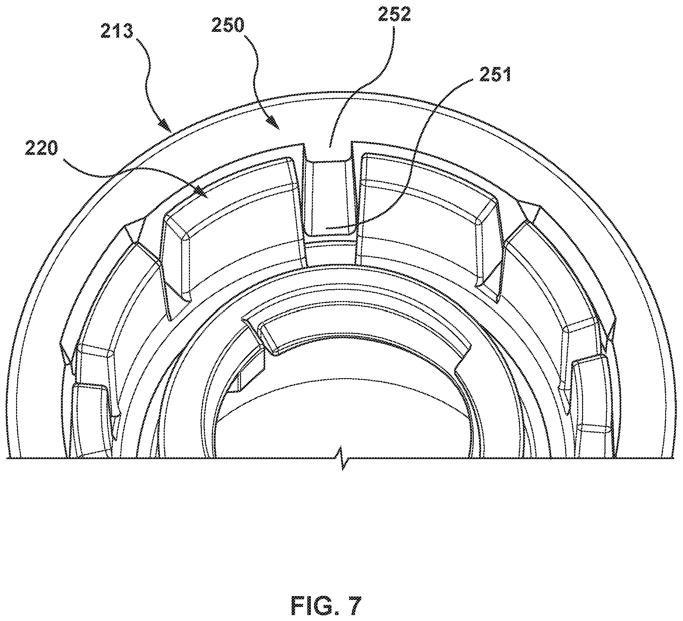

The introducer hub 210 of the expandable introducer 100 will now be described with reference to FIGS. 5A-11C. Referring to FIGS. 5A-5B, the introducer hub 210 includes, *inter alia,* a hub body 213, a sealcap 220, and a seal section 270. The hub body 213 includes a distal end 214, a proximal end 215, and a lumen or passageway 216 extending from the proximal end 215 to the distal end 214. The shaft portion 120 of the expandable introducer 100 attaches to the distal end 214 of the introducer hub 213 and extends distally therefrom. An outer surface of the hub body 213 tapers in a distal direction such that the distal end 214 of the hub body 213 has a first diameter smaller than a second diameter at the proximal end 215 of the hub body 213, as shown in FIG. 5A. In the embodiment shown, the first diameter of the introducer hub 213 is about 9-12 mm and the second diameter of the introducer hub 213 is about 25-30 mm.

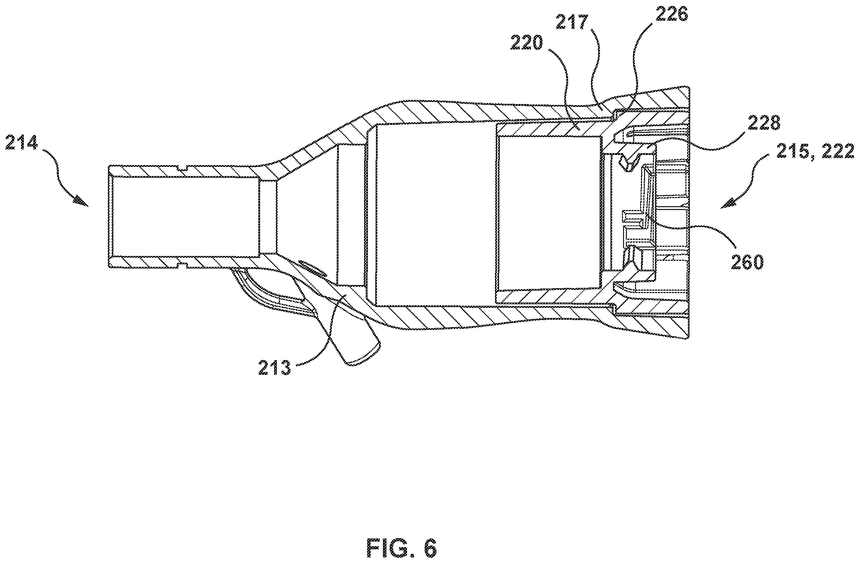

The sealcap 220 is a generally cylindrically-shaped piece of the introducer hub 210 that includes a body 225, a distal end 221, a proximal end 222, and a central lumen or passageway 224 extending from the proximal end 222 to the distal end 221, as shown in FIGS. 5A-5B. The proximal end 222 of the sealcap 220 includes a shoulder or lip 226 at an intersection of the body 225 and the proximal end 222 such that the body 225 has a smaller outer diameter than the proximal end 222. The outer diameter of the lip 226 of the sealcap 220 is about 25-30 mm and the outer diameter of the body 225 of the sealcap 220 is about 20-25 mm. The outer diameter of the body 225 is configured to fit with central lumen 216 of the hub body 213 with the lip 226 of the seal cap 220 abutting a corresponding lip or shoulder 217 of the hub body 213, as shown in FIG. 5B. The seal cap 220 further includes protrusions 227 at the proximal end 222 that mate with corresponding openings 218 in the hub body 213 to couple the seal cap 220 to the hub body 213 in a snap-fit connection. Further, when the sealcap 220 is coupled to the introducer hub 213, the distal end 221 of the sealcap 220 abuts the seal section 270 disposed within the introducer hub 213, maintaining compression in the seals and securing the seals in place. Further, in the embodiment shown, the proximal end 222 of the sealcap 220 aligns with the proximal end 215 of the hub body 213 when the sealcap 220 is secured within the hub body 213, as shown in FIG. 5B and FIG. 6.

The sealcap 220 further includes an internal flange 228 for mating with a catheter delivery system and/or a dilator hub, as explained in more detail below. An internal surface of the sealcap 220 further includes a locking mechanism 260 configured for attachment of a catheter delivery system, a valve relief, and/or a dilator hub to the seal cap 220, as explained in more detail below.

As shown in FIG. 5B, the longitudinal length of the sealcap 220 is such that it minimizes a maximum insertion angle α of a shaft 229 of a delivery catheter system, a valve relief, and/or a dilator assembly. The maximum insertion angle α is a measurement of the maximum angle at which the shaft 229 can enter, or be inserted into, the proximal end 222 of the sealcap 220. The maximum insertion angle α is measured from the central longitudinal axis CLA of the expandable introducer 100. In other words, the central longitudinal axis CLA denotes 0° when the maximum insertion angle α is being measured. By minimizing the maximum insertion angle α of the valve relief 230, the risk of damaging the seals in the seal section 270 is reduced. The maximum insertion angle α may be a function of several parameters, including but not limited to, the longitudinal length of the sealcap 220 and an inner diameter of the sealcap 220. In the embodiment shown, the longitudinal length of the sealcap 220 is about 16 mm, the inner diameter of the sealcap 220 is about 19-22mm, and the resulting maximum insertion angle α is about 5-6°.

Further, in some embodiments, as shown in FIGS. 6-7, the hub body 213 and sealcap 220 may include an over-torqueing prevention mechanism 250. In particular, the hub body 213 may include a fin or protrusion 252 extending inwardly towards the central longitudinal axis CLA. The sealcap 220 includes a corresponding slot 251 that mates with the protrusions 252. The over-torqueing mechanism 250 prevents a user from torqueing out the sealcap 220 when coupling a dilator hub or catheter delivery system to the sealcap 220, as described in more detail below.

As noted above, the introducer hub 210 includes a seal section 270 disposed within the central lumen 216 of the hub body 213, as can be seen in FIG. 5B. The seal section 270 is configured to house at least one seal within the introducer hub 213. The embodiment shown in FIG. 5B includes exactly three seals, which is described in further detail below.

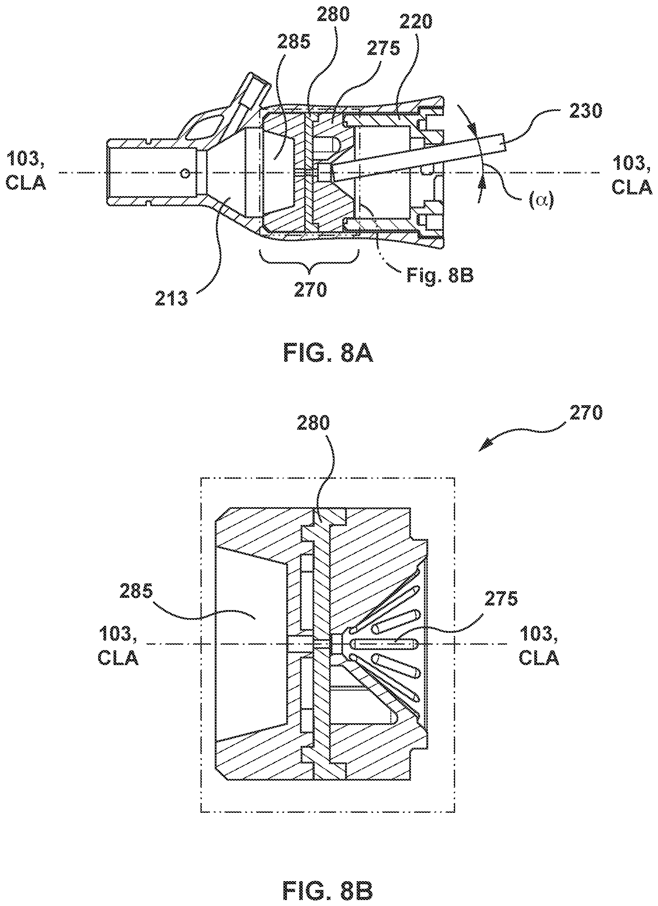

FIG. 8A shows the seal section 270 and the sealcap 220 disposed within the hub body 213 of the introducer hub 210. The seal section 270 is disposed distal to the sealcap 220. The seal section 270 houses at least one seal within the central lumen 213 of the hub body 213. The seal section 270 is configured to prevent blood within the shaft portion 120 of the expandable introducer 100 from leaking and to prevent air from entering the shaft portion 120 of the expandable introducer 100, and thus the vasculature of the patient, which prevents air embolism and excessive blood loss. In other words, the seal section is designed to maintain hemostasis and prevent blood flow out of a patient's vessel when the expandable introducer 100 is in use and exposed to the pressures of the arterial system. The seal section 270 also seal allows for low insertion and withdrawal forces of dilator, valve relief and delivery catheter system while maintaining a self-sealing valve with changes in arterial pressure.

In the embodiment shown, the seal section 270 includes a first or proximal seal 275, a second or middle seal 280, and a third or distal seal 285, as shown in FIGS. 8A-8B. However, this is not meant to be limiting. For example, the seal section 270 may include exactly one seal, or exactly two seals, or exactly four seals, or four or more seals. In the embodiment shown, the first seal 275 is the proximal-most seal and the third seal 285 is the distal-most seal. The second seal 280 is disposed between, and is coupled to, the first seal 275 and the third seal 285. It should be understood that the first seal 275, the second seal 280, and the third seal 285 maintain hemostasis when various devices are inserted through the seals and when all devices are removed from the seals.

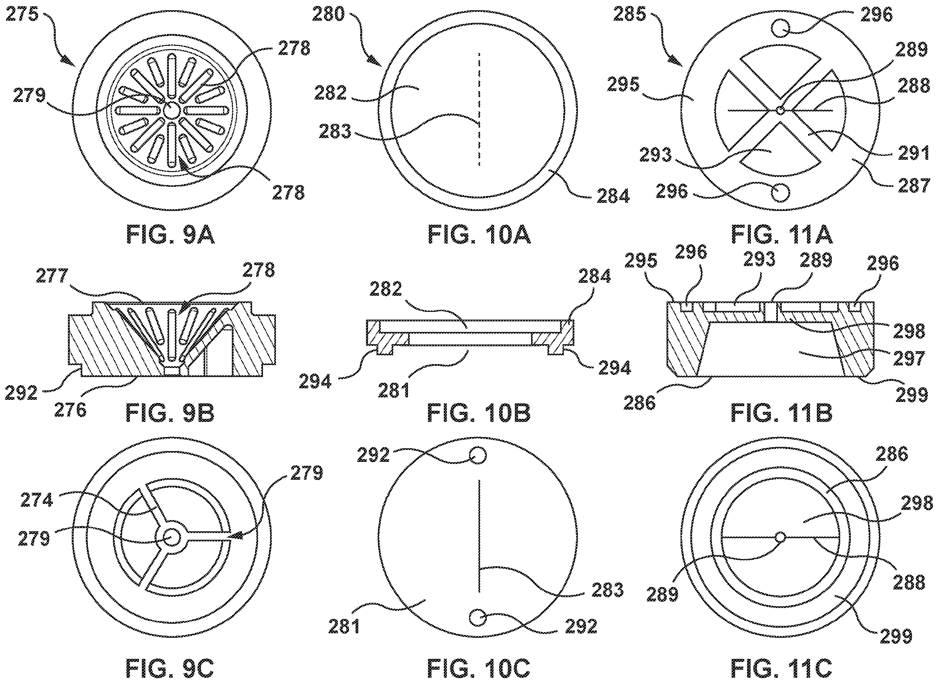

FIGS. 9A-9C show a proximal view, a side cross-sectional view, and a distal view, respectively, of the first seal 275 according to embodiments hereof. The first seal 275 includes a distal end 276, a proximal end 277, and a central passageway 279 extending from the proximal end 277 to the distal end 276. The central passageway 279 is co-axial with the central longitudinal axis CLA. The central passageway 279 is substantially conically shaped from the proximal end 277 to a substantially circular hole extending through the distal end 276. The first seal 275 has a conical shape that tapers towards the distal end 276 of the first seal 275, as shown in FIG. 9B. Thus, the central passageway 279 tapers from a larger diameter and the proximal end 277 to a smaller diameter at the distal end. An inner surface of first seal defining the central passageway 279 may include a plurality of protruding ribs 279, as shown in FIG. 9A. The plurality of protruding ribs 279 are configured to break surface friction on the interior surface and aid in retention of silicone oil to help reduce insertion forces when a dilator assembly (not shown), valve relief (not shown), or a delivery catheter system is inserted through the first seal 275. An exterior surface of the first seal 275 may include three supporting walls 274 supporting the conical portion of the first seal 275, as shown in FIG. 9C. The first seal 275 may comprise silicone, polyurethanes and/or other highly elastic polymers.

FIGS. 10A-10C show a proximal view, a side cross-sectional view, and a distal view, respectively, of the second seal 280 according to embodiments hereof. The second seal 280 includes a distal surface 281 and a proximal surface 282. The distal surface 281 of the second seal 280 abuts the third seal 285 and the proximal surface 282 of the second seal 280 abuts the first seal 275, as shown in FIGS. 8A-8B. A rim 284 extends proximally from the proximal surface 282 of the second seal 270, as shown in FIGS. 10A and 10B. The proximal rim 284 is configured to mate with a recess 292 in the distal end 277 of the first seal 275, as shown in FIG. 8B. Protrusions 292 extend distally from the distal surface 281 of the second seal 275, as shown in FIG. 10B and 10C. The protrusions 294 are configured to mate with corresponding recesses 296 in a proximal end of the third seal 285, as shown in FIG. 9B. The second seal 280 further includes a slit 283 extending through the proximal and distal surfaces 282, 281 thereof. The slit 283 extends a majority of a diameter between the protrusions 292 at the distal surface 281 and a corresponding length on the proximal surface 282. The slit extends through a about a center of the second seal 280 such that a portion of the slit 283 aligns with the central lumen 279 of the first seal and a central lumen 289 of the third seal 285. The slit 283 enables a dilator assembly, a valve relief, catheter delivery system shaft, and/or a guidewire to extend through the second seal 280 while maintaining hemostasis by sealing around the exterior surface of the device that extending through the second seal 280. The second seal 280 may comprise silicone, polyurethanes and/or other highly elastic polymers.

FIGS. 11A-11C show a proximal view, a side cross-sectional view, and a distal view, respectively, of the third seal 285 according to embodiments hereof. The third seal 285 includes a distal end 286, a proximal end 287, a central opening 289, a slit 288, and a cavity 297. The proximal end 287 of the third seal 285 abuts the second seal 280, as can be seen in FIGS. 8A-8B. The third seal 285 is generally cylindrically-shaped. The proximal end 287 of the third seal 285 includes a proximal surface 293 and a rim 295 extending proximally from the proximal surface 293 at the perimeter of the proximal surface 293. The rim 295 includes recesses 296 configured to receive the protrusion 292 in the second seal 280, as shown in FIG. 8B. The proximal end 287 further includes support ribs 291 extending proximally from the proximal surface 293. The proximal support ribs 291 are arranged in an X-pattern through a center of the proximal surface 293. The distal end 286 of the third seal 285 includes a distal surface 298 opposite the proximal surface 293. The distal end 286 further includes a rim 299 extending distally from the distal surface 298, with a cavity 297 defined by the distal surface 298 and an interior surface of the rim 299. A central opening 289 extends from the proximal surface 293 to the distal surface 298. More specifically, the central opening 289 in the embodiment shown extends from a center of the X-pattern of the ribs 291 to the distal surface 298.

The third seal 285 further includes a straight, thin slit 288 extending from the proximal surface 293 to the distal surface 298. The slit 288 extends through a majority of a diameter of the third seal 285. When the seals are arranged together in the seal section 270, the slit 288 of the third seal 285 is disposed perpendicular to the slit 283 of the second seal 280 such that the slits 283, 288 create a + (plus) shape that cross at a center of each slit 283, 288. The slit 288 allows a dilator assembly, a valve relief, catheter delivery system shaft, and/or a guidewire to extend through the third seal 285 while still maintaining hemostasis by sealing around the exterior surface of the device extending therethrough. The third seal 285 may comprise silicone, polyurethanes and/or other highly elastic polymers.

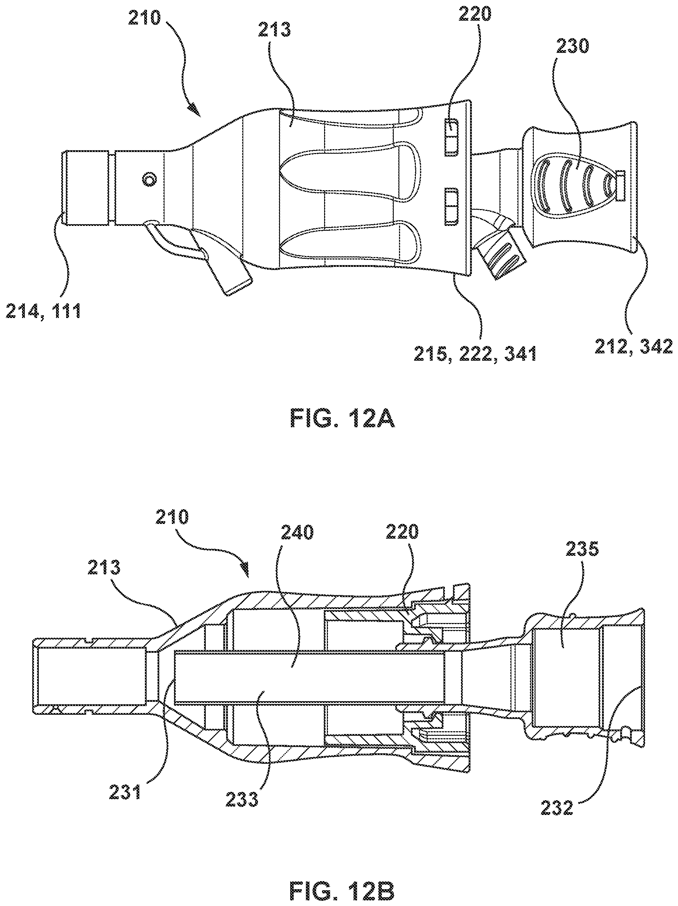

FIGS. 12A-12B show the introducer hub 210 with a valve relief 230 disposed win the central lumen 216 of the hub body 213. The valve relief 230 includes a hub 240 and a shaft 235 extending distally therefrom. The valve relief 230 includes a central passageway 233 extending from a proximal end 232 to a distal end 231 of the valve relief 230. The valve relief 230 is configured to receive a delivery catheter system therein such that the shaft 235 protects a valve prosthesis disposed on the delivery catheter system such that the valve prosthesis is not damaged when passing through the introducer hub 210, particularly through the seal section 270. Thus, the shaft 235 extends distally at least through the seal section 270 of the introducer hub 210. The hub 240 of the valve relief 230 includes an external thread or external threads to mate with the locking mechanism 260 of the sealcap 220, as explained in more detail below with respect to the dilator hub.

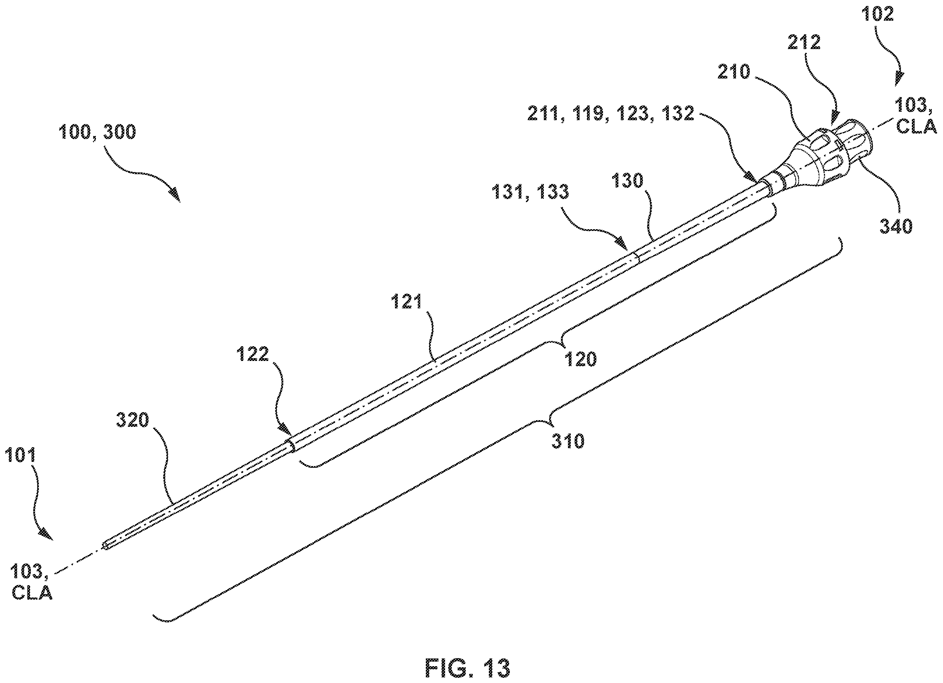

FIG. 13 shows the expandable introducer 100 in combination with a dilator assembly 300 to be used in medical procedures. The dilator assembly 300 includes a dilator shaft 310 and a dilator hub 340. The dilator shaft 310 includes a distal tip 320 and a proximal end coupled to the dilator hub 340. A central lumen 305 extends through the dilator hub 340 and the dilator shaft 310. A proximal end of the dilator shaft 310 couples to the dilator hub 340 and extends distally therefrom. The dilator shaft 310 is sized and shaped to advance distally through the expandable introducer 100 and the dilator hub 340 is configured to couple to the introducer hub 210 of the expandable introducer 100, as described in more detail below.

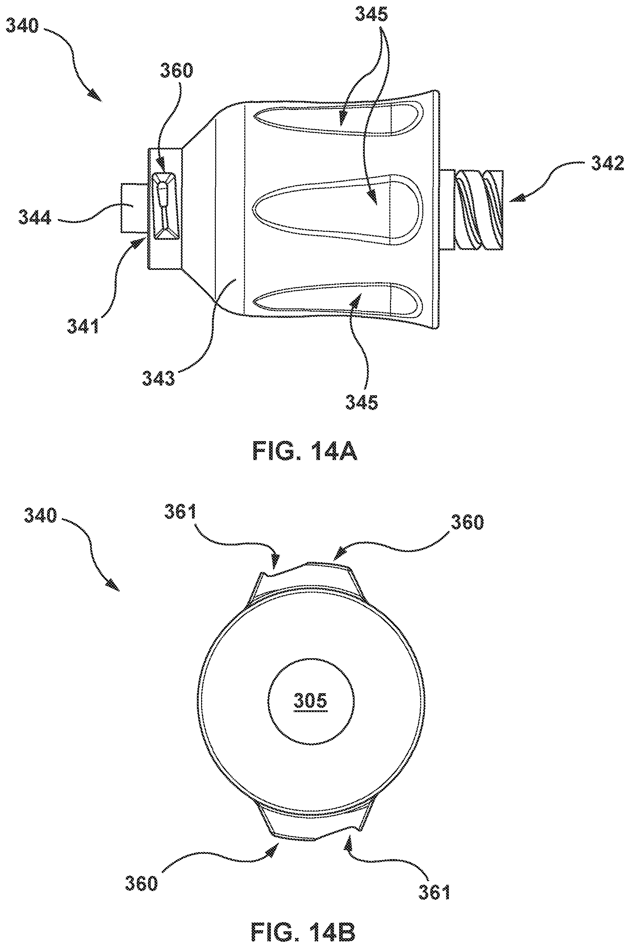

Referring to FIG. 14A, the dilator hub 340 a body 343, a distal end 341, and a proximal end 342. The distal end 341 of the dilator hub 340 couples to the sealcap 220, as described in more detail below. The body 343 may include indented grips 345 disposed on an exterior surface of thereof. The indent grips 345 are longitudinal cut-outs on the body 343 that allow a user to easily and securely grip the exterior surface of the dilator body 343. In the embodiment shown, the body 343 includes six indent grips 345 (only three are shown as only half of the exterior surface of the body 343 is shown), but this is not meant to be limiting, and more or fewer indent grips may be utilized.

Referring to FIG. 14B the distal end 341 of the dilator hub includes a pair of quarter-turn threads 360 configured to mate with the locking mechanism 260 of the sealcap 220, as described in more detail below. The threads 360 each include a notch adjacent thereto for haptic feedback of the interlock between the dilator hub and the sealcap 220.

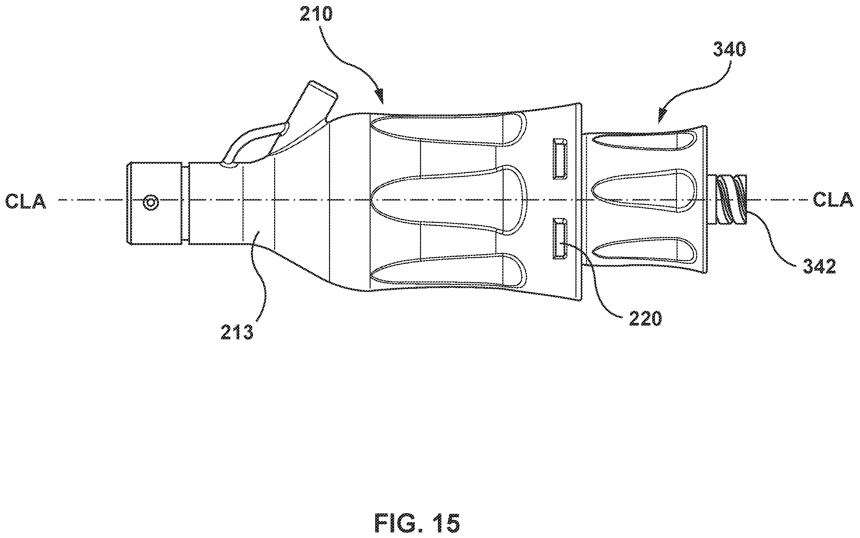

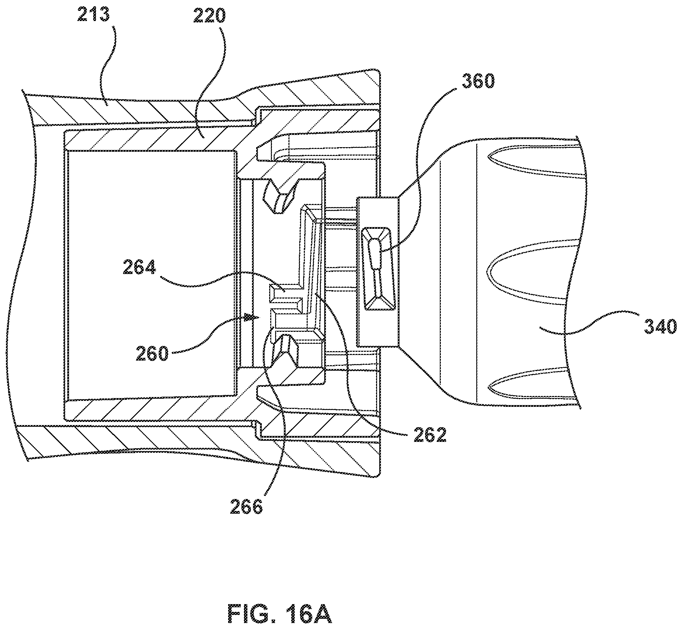

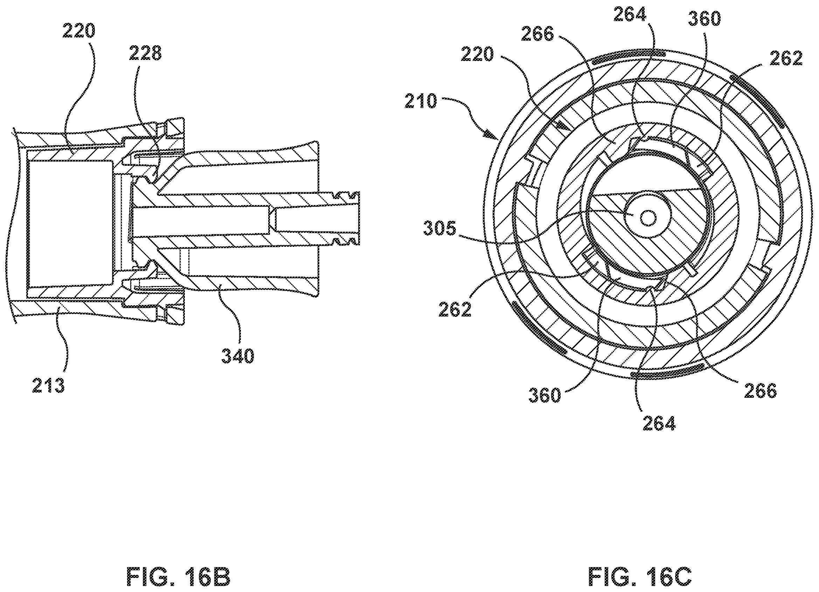

Referring to FIGS. 15 and 16A-16C, the dilator hub 340 is shown coupled to the sealcap 220, which is coupled to the hub body 213 of the introducer hub 210. In particular, FIG. 16A shows the dilator hub 340 entering the sealcap 220 but not yet coupled thereto. As shown in FIG. 16A, the interior surface of the sealcap 220 includes the locking mechanism 260, which includes two internal threads or grooves 262, two tactile feedback bumps 264, and two hard stops 266. As shown in FIG. 16C, each internal thread 262, tactile feedback bump 264, and hard stop 266 is disposed above 180 degrees around an internal circumference of the seal cap 220 as the other of the internal thread, tactile feedback bump 264, and hard stop 266, respectively. Each one of the external threads 360 of the dilator hub 340 is inserted into a corresponding one of the internal threads 262. The dilator hub 340 is rotated quarter turn to secure the dilator hub 340 to the seal cap 220. The tactile feedback bumps 264 provides tactile feedback to the user when the corresponding external thread 360 basses the bump 264 such that the bump 264 settles into the corresponding haptic feedback notch 361. The hard stops 266 prevent the dilator hub 340 from being over-rotated. Although the embodiment described includes two internal and external threads for a quarter-turn connection between the dilator hub 340 and the sealcap 220, this is not meant to be limiting, and additional threads or differing amounts of turn may be utilized.

As used herein, the term "generally" and "substantially" mean approximately. When used to describe angles such as "substantially parallel" or "substantially perpendicular" the term "substantially" means within 10 degrees of the angle. When used to describe shapes such as "substantially" or "generally" cylindrical or "substantially" or "generally" tube-shaped or "generally" or "substantially" conical, the terms mean that the shape would appear cylindrical or tube-shaped or conical to a person of ordinary skill in the art viewing the shape with a naked eye. The term "about" as used herein to refer to dimensions means within 5% of the dimension.

It should be understood that various embodiments disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g, all described acts or events may not be necessary to carry out the techniques). In addition, while certain aspects of this disclosure are described as being performed by a single device or component for purposes of clarity, it should be understood that the techniques of this disclosure may be performed by a combination of devices or components.

Further disclosed herein is the subject matter of the following clauses:
1. An expandable introducer (100) comprising:
   a hub (210) having a distal end (211) and a proximal end (212); and
   a tubular sheath (121) coupled to the hub (210) and extending distally therefrom, the tubular sheath (121) defining a central lumen (103), wherein a circumference of the tubular sheath (121) includes a thick wall region (162) having a first wall thickness and a thin wall region (161) having a second wall thickness smaller than the first wall thickness, wherein the thin wall region (161) is configured to be folded such that the tubular sheath (121) includes an unexpanded, folded state and is configured to expand to an expanded, unfolded state in response to a device passing though the central lumen, wherein a first outer diameter of the tubular sheath (121) in the unexpanded, folded state is smaller than a second outer diameter of the tubular sheath (121) in the expanded, unfolded state.
2. The expandable introducer (100) of clause 1, wherein the first wall thickness is about 0.4-0.7mm.
3. The expandable introducer (100) of clause 1 or clause 2, wherein the second wall thickness is about 0.05-0.2mm.
4. The expandable introducer (100) of any one of clauses 1-3, wherein:
   the thick wall region (162) of the tubular sheath (121) includes an outer wall (126) having a first layer (127) and a second layer (128), and a liner (129) coupled to an inner surface of the outer wall (126), the liner (129) defining the central lumen (103)of the tubular sheath (121); and
   the thin wall region (161) of the tubular sheath (121) includes the liner (129) and only the second layer (128) of the outer wall (126).
5. The expandable introducer (100) of clause 4, wherein the second layer (128) in the thick wall region (162) has a third thickness, and the second layer (128) in the thin wall region (161) has a fourth thickness smaller than the third thickness.
6. The expandable introducer (100) of any one of clauses1-5, wherein the tubular sheath (121) comprises a proximal end (123), wherein the proximal end (123) of the tubular sheath (121) does not include an unexpanded, folded configuration.
7. The expandable introducer (100) of any one of clauses 1-6, further comprising a hemostatic jacket (130) surrounding at least a proximal end (123) of the tubular sheath (121), wherein the hemostatic jacket (130) is configured to prevent blood flow from outside of the tubular sheath.
8. The expandable introducer (100) of clause 7, wherein the hemostatic jacket (130) is radially expandable.
9. The expandable introducer of (100)clause7 orclause8, wherein the hemostatic jacket (130) is about 10 - 12 cm in length.
10 The expandable introducer (100) of clause7 or clause8, wherein the hemostatic jacket (130) is about 30 -35 cm in length.
11. The expandable introducer (100) of any one of clauses 7-10, wherein the hemostatic jacket (130) is heat bonded to the proximal end (123) of the tubular sheath (121).
12. The expandable introducer (100) of any one ofclauses7-11, wherein the hemostatic jacket (130) includes an angled tip.
13. The expandable introducer (100) of any one of clauses 7-12, wherein the hemostatic jacket (130) is attached to only the thick wall region (162) of the tubular sheath (121).
14. The expandable introducer (100) of any one of clauses 1-13, further comprising a tip (140) coupled to a distal end (122) of the tubular sheath (121) and extending distally from the distal end (122) of the tubular sheath (121).
15. The expandable introducer (100) of clause 14, wherein the tip (140) comprises a weakened region configured to tear to allow the tip (140) to radially expand in response to a device being advanced through the tip (140).
16. The expandable introducer (100) of clause 15, wherein the tip (140) includes an axial slit, wherein circumferential edges at proximal and distal ends of the longitudinal are attached to each other, and wherein the weakened region comprises the axial slit between the proximal and distal attachments.
17. The expandable introducer (100) of clause 15, wherein the weakened region comprises a longitudinal perforation.
18. The expandable introducer (100) of any one of clauses 1-17, wherein the tubular sheath (121) is shape set to return to the unexpanded, folded state after the device has passed through to the central lumen (103).
19. The expandable introducer (100) of any one of clauses 1-18, wherein the first outer diameter of the tubular sheath (121) is about 5-7mm.
20. The expandable introducer (100) any one of clauses 1-19, wherein the second outer diameter of the tubular sheath (121) is about 8-10 mm.
21. An introducer hub (210) comprising:
   a hub body (213) having a proximal end (214), a distal end (215), and a central lumen (216);
   a seal section (270) disposed within the central lumen (216) of the hub body (213), the seal section (270) including at least one seal; and
   a sealcap (220) coupled to the hub body (213) and at least partially disposed within the central lumen (216), the sealcap (220) having a distal end abutting the seal section (270), the sealcap (220) including a locking mechanism (260) on an internal surface thereof configured to interlock with a device to be inserted into the introducer hub (210).
22. The introducer hub (210) of clause21, wherein the locking mechanism (260) includes an internal quarter-turn thread (262), a haptic feedback bump (264), and a hard-stop (266).
23. The introducer hub (210) of clause 21 or clause 22, wherein:
   the sealcap (220) includes a plurality of protrusions (227) extending from an outer surface thereof;
   the hub body (213) includes a plurality of openings (218); and
   the plurality of protrusions (227) are configured to mate with plurality of openings (218) to couple the sealcap (220) to the hub body (213) in a snap-fit connection.
24. The introducer hub (210) of wherein the plurality of protrusions (227) are disposed on the proximal end of the sealcap (220) and the plurality of openings (218) are disposed on the proximal end of the hub body (213).
25. The introducer hub (210) of any one of clauses21-24, wherein the sealcap (220) has a longitudinal length of about 14-16 mm, and internal diameter of about 19-22mm, and wherein a maximum insertion angle of a shaft that can pass through the seal section is 5-6°.
26. The introducer hub (210) of any one of clauses21-25, wherein the seal section (270) comprises a first seal (275), a second seal (280), and a third seal (285)
27. The introducer hub (210) of clause26, wherein the first seal (275) comprises a central passageway (279) that is substantially conically-shaped tapering from a proximal end (277) of the first seal (275) to a substantially circular hole extending through a distal end (276) of the first seal (275).
28. The introducer hub (210) of clause26 orclause 27, wherein the second seal (280) is disposed distal to the first seal (275) and comprises a slit (283) including a central portion that aligns with the hole of the first seal (275), wherein the slit (283) enables the device to be inserted into the introducer hub (210) extend through the second seal (280) while maintaining hemostasis by sealing around an exterior surface of the device.
29. The introducer hub (210) of any one of clauses26-28, wherein the third seal (285) is disposed distal of the second seal (280) and comprises a central opening (289), a slit (288), and a cavity (297).
30. The introducer hub (210) of clause29, wherein the third seal (285) includes a distal surface (298) and a proximal surface (293) opposite the distal surface (298), wherein the central opening (289) extends from the proximal surface (293) to the distal surface (298) and the slit (288) extends from the proximal surface (293) to the distal surface (298).
31. The introducer hub of clause 30, wherein the slit (288) of the third seal (285) is perpendicular to the slit (283) of the second seal (280).

## Claims

1. An expandable introducer (100) comprising:
a hub (210) having a distal end (211) and a proximal end (212); and
a tubular sheath (121) coupled to the hub (210) and extending distally therefrom, the tubular sheath (121) defining a central lumen (103), wherein a circumference of the tubular sheath (121) includes a thick wall region (162) having a first wall thickness and a thin wall region (161) having a second wall thickness smaller than the first wall thickness, wherein the thin wall region (161) is configured to be folded such that the tubular sheath (121) includes an unexpanded, folded state and is configured to expand to an expanded, unfolded state in response to a device passing though the central lumen, wherein a first outer diameter of the tubular sheath (121) in the unexpanded, folded state is smaller than a second outer diameter of the tubular sheath (121) in the expanded, unfolded state.

2. The expandable introducer (100) of claim 1, wherein the first wall thickness is about 0.4-0.7mm.

3. The expandable introducer (100) of claim 1 or claim 2, wherein the second wall thickness is about 0.05-0.2mm.

4. The expandable introducer (100) of any one of claims 1-3, wherein:
the thick wall region (162) of the tubular sheath (121) includes an outer wall (126) having a first layer (127) and a second layer (128), and a liner (129) coupled to an inner surface of the outer wall (126), the liner (129) defining the central lumen (103)of the tubular sheath (121); and
the thin wall region (161) of the tubular sheath (121) includes the liner (129) and only the second layer (128) of the outer wall (126); and optionally wherein the second layer (128) in the thick wall region (162) has a third thickness, and the second layer (128) in the thin wall region (161) has a fourth thickness smaller than the third thickness.

5. The expandable introducer (100) of any one of claims 1-4, wherein the tubular sheath (121) comprises a proximal end (123), wherein the proximal end (123) of the tubular sheath (121) does not include an unexpanded, folded configuration.

6. The expandable introducer (100) of any one of claims 1-5, further comprising a hemostatic j acket (130) surrounding at least a proximal end (123) of the tubular sheath (121), wherein the hemostatic jacket (130) is configured to prevent blood flow from outside of the tubular sheath.

7. The expandable introducer (100) of claim 6, wherein the hemostatic jacket (130) is radially expandable.

8. The expandable introducer of (100) claim 6 or claim 7, wherein the hemostatic jacket (130) is about 10 - 12 cm in length; or wherein the hemostatic jacket (130) is about 30 -35 cm in length.

9. The expandable introducer (100) of any one of claims 6-8, wherein the hemostatic jacket (130) is heat bonded to the proximal end (123) of the tubular sheath (121).

10. The expandable introducer (100) of any one of claims 6-9, wherein the hemostatic jacket (130) includes an angled tip; and/or wherein the hemostatic jacket (130) is attached to only the thick wall region (162) of the tubular sheath (121).

11. The expandable introducer (100) of any one of claims 1-10, further comprising a tip (140) coupled to a distal end (122) of the tubular sheath (121) and extending distally from the distal end (122) of the tubular sheath (121).

12. The expandable introducer (100) of claim 11, wherein the tip (140) comprises a weakened region configured to tear to allow the tip (140) to radially expand in response to a device being advanced through the tip (140).

13. The expandable introducer (100) of claim 12, wherein the tip (140) includes an axial slit, wherein circumferential edges at proximal and distal ends of the longitudinal are attached to each other, and wherein the weakened region comprises the axial slit between the proximal and distal attachments; or wherein the weakened region comprises a longitudinal perforation.

14. The expandable introducer (100) of any one of claims 1-13, wherein the tubular sheath (121) is shape set to return to the unexpanded, folded state after the device has passed through to the central lumen (103).

15. The expandable introducer (100) of any one of claims 1-14, wherein the first outer diameter of the tubular sheath (121) is about 5-7mm; and/or wherein the second outer diameter of the tubular sheath (121) is about 8-10 mm.
